# NEUE EUROPÄISCHE PATENTSCHRIFT

(11) **EP 0 911 032 B2**
(45) Veröffentlichungstag und Bekanntmachung des Hinweises auf die Entscheidung über den Einspruch: **01.09.2004**
(45) Hinweis auf die Patenterteilung: 13.03.2002
(21) Anmeldenummer: 97118648.1
(22) Anmeldetag: 27.10.1997
(51) Int. Cl.: A61K 35/78

(54) **Brausezubereitung mit Pflanzenextrakt**
Effervescent compositions containing plant extracts
Compositions effervescentes contenant des extraits végétaux

(43) Veröffentlichungstag der Anmeldung: 28.04.1999
(73) Patentinhaber: Dr. Gergely & Co., 1053 Wien (AT)
(72) Erfinder: Gergely, Gerhard, Dr., 1053 Wien (AT); Gergely, Imgard, 1053 Wien (AT); Gergely, Thomas, Dr., 1053 Wien (AT); Gergely, Stefan, Dr., 1053 Wien (AT)
(74) Vertreter: Büchel, Kurt F.

(56) Entgegenhaltungen:
- WO-A-90/03179
- WO-A-96/29085
- DATABASE WPI Week 9739 Derwent Publications Ltd., London, GB; AN 97-424658 [39] XP002060147 & WO 97 29642 A (E. MAS CRIADO,ES) 21.August 1997
- Technologie pflanzlicher Arzneimittelbereitungen; List, Schmidt, Wissenschaftliche Verlagsgesellschaft mbH Stuttgart 1984, Kapitel 7.1 "Herstellung fester Arzneiformen aus Trockenextrakten"
- Encyclopedia of Pharmaceutical Technology, Vol. 16, Marcel Dekker, Inc., Ed. J. Swarbrick, J. C. Boylan Inc., 1997, Kapitel "Waxes", S. 335-361

## Beschreibung

Die Erfindung betrifft eine Brausezubereitung nach dem Oberbegriff des Anspruches 1. Pflanzenextrakte waren seit jeher eine beliebte Darreichungsform zur Prophylaxe und Therapie; sie gewinnen nicht zuletzt durch die zunehmende Aufklärung des Wirkmechanismus und der Struktur der wirksamen Inhaltsstoffe weiter an Bedeutung. Für erforderliche höhere Dosierungen ist der Instant-Tee bisher die wesentlichste Darreichungsform, die aber einige Nachteile hat, nämlich einerseits die unexakte Dosierung durch das Auflösen eines Tee- oder Esslöffels des Instant-Tees in Wasser, wobei die Füllmenge kaum exakt eingehalten werden kann; andererseits sind die - üblicherweise in Pulveroder Granulatform vorliegenden - pflanzenextrakthaltigen Instant-Tees sehr hygroskopisch und ballen bzw. verklumpen im Behälter, nachdem dieser einige Male geöffnet wurde.

Um diese Nachteile zu vermeiden und um eine attraktive Darreichungsform zu finden, wurde die Verabreichung der Pflanzenextrakte in Form einer Brausetablette entwickelt. Dabei hat es sich aber gezeigt, dass speziell die sehr leicht wasserlöslichen Pflanzenextrakte, die meistens in einer - aus wässriger und/oder alkoholischer Lösung heraus sprühgetrockneten - feinpulverigen Form vorliegen, die Auflösung der Brausetablette extrem verlangsamen. Dazu gehören u.a. Efeu- und Sabale-Trockenextrakte, Solidago-, Plantago-, Brennesselwurzel-, Brennesselblätter-, Birkenblätter-, Cynara- und Thymian-Extrakte, Extrakte von Johanniskraut, Harpagophytum, Gingko, sowie Ruscus (Extractum Rusci aculeati). Das Verlangsamen der Auflösung entsteht dadurch, dass diese sehr leicht löslichen Pflanzenextrakte bei Zutritt von Wasser sehr schnell hochkonzentrierte, klebrige, schleimige Lösungen bilden, die den weiteren Wasserzutritt in das Innere der Tablette verhindern, wodurch eine rasche Auflösung behindert wird. Überdies werden auch Brauseteilchen, die den Zerfall und das Auflösen der Tablette bewirken sollen, von dieser Lösung umhüllt und damit ihre Reaktion mit dem Wasser unterbunden. Hinzu kommt noch, dass die meisten Pflanzenextrakte saponinhältig sind und folglich ein extremes Schaumverhalten in der Brausetablette zeigen. Dadurch wird die Auflösung weiter gebremst, weil zwischen den Brauseteilchen schaumige, hochkonzentrierte Lösungen entstehen.

So resultieren z.B. beim Zufügen einer Dosis von 400 mg Solidagoextrakt zu einem Brausegranulat, das anschliessend zu Tabletten verpreßt wird, beim Auflösen in Wasser Auflösezeiten von 15 bis 20 Minuten. Auch geringere Mengen, z.B. 65 mg eines Efeu-Trockenextraktes in einer Brausetablette, zeigen bei der Auflösung in 17°C warmem Wasser Auflösezeiten von 5 bis 7 Minuten und mehr. Dies entspricht nicht mehr den Anforderungen der Pharmakopöen, gemäss welcher die Auflösezeit von Brausetabletten in Wasser von 20°C weniger als 5 Minuten betragen soll.

Mit gängigen Massnahmen, wie z. B. durch das Zufügen von Anti-Schaummitteln, wie z. B. Simethicon oder Dimethicon, oder durch das Aufbringen des Anti-Schaummittels auf den getrockneten Pflanzenextrakt selbst, konnte nur eine geringfügige Verbesserung durch Verringerung des Schäumens erreicht werden, und - damit einhergehend - auch nur eine geringe Verkürzung der Auflösezeit der Brausetablette.

Auch eine Granulierung der Pflanzenextrakte mit dem Ziel, die Teilchen zu vergrössern und ihre Anzahl innerhalb der Brausetablette zu verringern, um so die Entstehung einer konzentrierten Lösung rund um die Pflanzenextrakt- und Brauseteilchen herum beim Auflösen der Tablette in Wasser zu verhindern, war nicht zielführend, da dann eben diese granulierten Teilchen extrem zusammenklebten und unerwünschte und keinesfalls schnelle Auflösungseigenschaften zeigten.

Auch die Anbindung an eine schwer lösliche Substanz führte nicht zu einer schnellen Auflösezeit, da die Extrakte an der Oberfläche der schwer löslichen Substanz ebenfalls hochkonzentrierte Lösungen bildeten und somit diese Massnahme unwirksam machten.

Die Granulierung mit schwer löslichen Substanzen wiederum führte dazu, dass die Brausetablette mit Pflanzenextrakten zwar etwas schneller löslich wurde, jedoch die granulierten Teilchen sich nicht vollständig auflösen konnten und einen Rückstand bildeten.

Ziel der Erfindung war daher, eine galenische Zubereitung für leicht lösliche Pflanzenextrakte in Form einer Brausetablette zu schaffen, die eine Auflösezeit von unter 4 Minuten und eine signifikant reduzierte Schaumbildung aufweist.

Die Lösung dieser Aufgabenstellung und die Überwindung der zuvor erwähnten Probleme wurde überraschend durch die im Kennzeichen des Anspruches 1 beschriebenen Massnahmen erstmalig möglich. Besondere Ausführunqsformen und Weiterentwicklungen des Erfindungsgedankens sind in den Kennzeichen der abhängigen Ansprüche beschrieben.
Nun hat zwar die WO 96/29085 bereits eine Brausezusammensetzung mit Gingko-Biloba Trockenextrakt vorgeschlagen, die auch Macrogol-glycero-hydroxystearat enthält. Diese Mischung kann die erfindungsgemässe Aufgabenstellung nicht erfüllen. Die WO 96/29085 hatte auch eine ganz andere Aufgabenstellung, nämlich die Vermeidung von Abbauprozessen, und die Erzielung einer Stabilität der Lösung von einer Stunde. Dies wird dort durch einen Carbonat- bzw. Hydrogencarbonatüberschuss in der Brausezubereitung und einen daraus resultierenden pH-Wert der Lösung bzw. Suspension von 6-8 erzielt.

Die Erfindung beruht darauf, den Pflanzenextrakt mit zumindest einerfettigen, öligen oderwachsartigen Substanz zu behandeln, insbesondere einen dünnen Überzugsfilm dieser Substanz oder Substanzen auf den Pflanzenextraktteilchen zu erzeugen, wobei dieser hydrophoben Extrakt-Phase mit fettigen, öligen oder wachsartigen Substanzen bevorzugt auch Emulgatoren zugesetzt werden. Die fettigen, öligen oder wachsartigen Substanzen können entweder in geschmolzenem Zustand oder aufgelöst in einem Lösungsmittel, in dem auch die Emulgatoren löslich sind, auf die Pflanzenextraktteilchen aufgebracht werden. Die auf diese Weise behandelten Pflanzenextrakte können dann - gegebenenfalls unter weiterem Emulgatorzusatz - einem Brausegranulat zugemischt und die Mischung zu Tabletten verpreßt werden. Man erhält Brausetabletten mit Pflanzenextrakten, die sich in Wasser bei 17°C innerhalb von 1 ½ bis 4 Minuten auflösen

Durch die Behandlung mit den oben erwähnten Substanzen werden die Pflanzenextrakte in genügendem Masse hydrophob, um die Brausetablette bei der Auflösung in Wasser nicht zu verkleistern, so dass die Wirksamkeit der Brauseteilchen bei Zutritt von Wasser wunschgemäss zum Tragen kommt und dadurch eine schnelle Auflösung der Tablette ermöglicht wird. Durch den Kontakt mit dem Wasser entfalten die Brauseteilchen ihre Brauseaktivität und die Pflanzenextraktteilchen werden aus der Tablette - bedingt durch die hydrophobe Struktur aufgrund des Brauseeffekts herausgeschleudert und lösen sich erst anschliessend auf. Dabei hat es sich als vorteilhaft erwiesen, der Phase mit den fettigen, öligen oder wachsartigen Substanzen geeignete Emulgatoren zuzusetzen, um entsprechende Suspensionseigenschaften für diese Substanzen beim Auflösen der Brausetablette in Wasser zu erzielen.

Als fettige, ölige oder wachsartige Substanzen, die zur Oberflächenbehandlung der Pflanzenextraktteilchen geeignet sind, können insbesondere folgende Substanzen eingesetzt werden: Ester mittelkettiger, pflanzlicher Fettsäuren, wie z. B. Capryl- und Caprinsäure, mit Glycerin oder Propylenglycol, vorzugsweise Miglyol® -Neutralöle.

Als Fettkomponenten können weiters alle essbaren, tierischen und pflanzlichen Fette eingesetzt werden. Es sind dies vor allem Triglyceride, die im wesentlichen aus Gemischen von Glycerinestern höherer Fettsäuren, insbesondere pflanzlichen oder tierischen Ursprungs, mit einer Kettenlänge von etwa 10 bis 22 C-Atomen, bestehen. Darunter fallen beispielsweise mikrokristalline Triglyceride und Glycerinester gesättigter, geradliniger und unverzweigter Fettsäuren, z.B. Glyceryl-Trimyristate, Glyceryl-Tripalmitate, Trimyristin, etc. Weitere geeignete Fettkomponenten sind z.B. Kokosfett, gehärtetes Kokosfett, hydrierte Rizinusöle, Tocopherolacetat, Ester höherer Fettsäuren wie z.B. Isopropylpalmitat, Polyethylenglykole, wie z.B. Carbowax® , wobei je nach Pflanzenextrakt Carbowax® 400 oder auch Carbowax® 6000 eingesetzt werden kann.

Die fettigen, öligen oder wachsartigen Substanzen werden in einer Menge von 0,5 bis 25 Gewichtsteilen, bevorzugt 0,8 - 19 Gewichtsteilen, bezogen auf 100 Gewichtsteile des leicht löslichen Pflanzenextraktes eingesetzt, wobei diese Substanzen wenigstens teilweise in der Pflanzenextrakt-Phase enthalten sind, aber auch zusätzlich als eigene Fettphase zum Brausegranulat zugefügt werden können. Dabei werden die Emulgatoren einerseits zusammen mit den fettigen, öligen oder wachsartigen Substanzen direkt auf den Pflanzenextrakt aufgebracht und gegebenenfalls andererseits in Form einer Phase zugefügt, wobei die Emulgatoren mit geeigneten Lösungsmitteln auf einen Träger aufgebracht werden, das Lösungsmittel abgedampft und diese Emulgatorphase zur Brausetablette zugemischt wird.

In Abhängigkeit von den eingesetzten fettigen, öligen oder wachsartigen Substanzen können verschiedenste Emulgatoren Verwendung finden. Empfehlenswert sind Phospholipide wie Lecithin, Metharin, Epikuron, weiters auch Polysorbate (Sorbitan-monolaurat, etc.) sowie ethoxylierte Glycerin-Fettsäureester (z.B. Tagate® ), Zuckerester, Glycerin-Polyethylenglykol-Oxystearat (Cremophor RH 40), Macrogof-Glycerolrizinoleat oder auch Natriumstearyllactat. Es können auch Benetzungsmittel, wie Natriumdioctylsulfosuccinat oder Natriumlaurylsulfat eingesetzt werden. Es können ein oder mehrere Emulgatoren in die Pflanzenextrakt-Phase eingearbeitet und/oder zusätzlich in einer weiteren Emulgator-Phase zur Endmischung für das pressfertige Granulat zugesetzt werden. Die Emulgatormenge beträgt zwischen 0,2 und 10, bevorzugt 0,3 - 8 Gewichtsteile bezogen auf 100 Gewichtsteile des eingesetzten, leicht löslichen Pflanzenextraktes, wobei auch einige Emulgatoren durch ihre ölige Eigenschaft die Wirksamkeit der fettigen Substanzen verstärken, bzw. einige Lipide auch Emulgator-Charakter haben, wie Propylenglycolstearat, Glycerinoleat, -laurat und -stearat..

Um die mitfettigen, öligen oderwachsartigen Substanzen behandelten Extrakte bei Bedarf besserfliessfähig zu machen, kann ein feiner Füllstoff zugesetzt werden, der sich an die fettige Oberfläche anhängt und so das Zusammenballen der Wirkstoff-Phase verhindert. Dafür kommen alle gängigen pharmazeutischen Tablettenfüllstoffe in Frage, wie Zuckeralkohole, Mannitol, Sorbitol, weiters Maltodextrin, pulverisierte Saccharose, pulverisierte Lactose, Fructose, Glucose, etc. Diese Füllstoffe können direkt in die Pflanzenextrakt-Phase eingebracht werden (siehe Beispiel 1 ), oder die Pflanzenextrakt-Phase wird nach der Herstellung mit einem Füllstoff homogen vermischt und anschliessend zum Brausegranulat mit den restlichen Inhaltsstoffen, Aromen etc. zugemischt.

Z. B. nimmt Mannitol oder Sorbitol einen Teil der öligen, fettigen oder wachsartigen Substanz sowie auch die Emulgatoren auf und verhindert das Zusammenklumpen der beschichteten Körner der Pflanzenextrakt-Phase. Im übrigen sind die erwähnten Füllstoffe auch als Träger für den bzw. die Emulgatoren, für das bzw. die Anti-Schaummittel, sowie für allfällige zusätzliche Mengen der öligen, fettigen oder wachsartigen Substanz geeignet, insbesondere dann, wenn die Aufnahmefähigkeit der Pflanzenextraktteilchen für diese Substanzen an der Oberfläche nicht ausreicht, um den optimalen Effekt zu erzielen.

Die Herstellung der Pflanzenextrakt-Phase kann folgendermassen erfolgen: Die leicht löslichen Pflanzenextrakte werden auf 45 bis 60°C angewärmt; eine Lösung oder Schmelze der fettigen, öligen oder wachsartigen Substanzen - vorzugsweise mit einem oder mehreren Emulgatoren - wird aufgebracht. Diese Lösung lässt man gleichmässig unter Rühren verteilen und verdampft anschliessend das Lösungsmittel, bevorzugt mittels Vakuum. Vor dem Trocknen können noch die Füllstoffe zugesetzt werden.

Um die Schaumbildung zu verringern, muss in den meisten Fällen, jedoch nicht zwingend, ein Anti-Schaummittel zugesetzt werden, das einerseits in die Fett-Emulgator-Phase eingebracht werden kann, andererseits aber auch als getrennte Phase zu einer Mischung des Brausegranulates und der Pflanzenextrakt-Phase zugesetzt werden kann, wobei das Anti-Schaummittel auf einen neutralen Hilfs-bzw. Füllstoff aufgezogen wird. Zur Herstellung der Anti-Schaummittel-Phase wird das Anti-Schaummittel mittels eines Lösungsmittels oder einer wässrigen Suspension auf einen Füllstoff aufgezogen; das Lösungsmittel wird abgedampft, und diese Phase wird der Tablette zugefügt.

Die Menge des eingebrachten Anti-Schaummittels, bezogen auf 100 Gewichtsteile des Pflanzenextraktes, kann zwischen 0 und 10 Gewichtsteilen betragen, d.h., dass bei gering saponinhaltigen, leicht löslichen Pflanzenextrakten in speziellen Fällen der Einsatz eines Anti-Schaummittels nicht erforderlich ist.

Es ist jedoch auch möglich, die ölige, fettige bzw. wachsartige Substanz, sowie die gegebenenfalls vorgesehenen Emulgatoren und/oder Anti-Schaummittel bereits der für die Herstellung des Pflanzenextraktes zur Sprühtrocknung vorgesehenen Lösung zuzusetzen.

Für die Herstellung der Brausebasis oder des Brausegranulates können alle gängigen Brausebestandteile Verwendung finden, wobei bevorzugt die Säurekomponente aus Zitronensäure, Weinsäure, Äpfelsäure, bzw. aus deren Salzen, wie z. B. MononatriumcitratoderMononatriumtartrat, besteht. Der Basenanteil der Brausebasis besteht zweckmässigerweise aus CO₂-abspaltenden Alkalibicarbonaten oder -carbonaten, wie Natrium- und/oder Kaliumhydrogencarbonat oder -carbonat, sowie teilweise, aber nicht ausschliesslich aus Erdalkalicarbonaten, wie Calciumcarbonat und/oder Magnesiumcarbonat.

Als Zusatzstoffe und Hilfsstoffe können insbesondere Süssstoffe, wie Zucker, Natriumcyclamat, Saccharin-Natrium, Aspartam, Acesulfam, sowie Geschmacksstoffe oder andere galenische Füllstoffe, wie Zuckeralkohole, z.B. Mannitol und Sorbitol, sowie auch Maltodextrin, gegebenenfalls Saccharose, Fructose, Lactose, etc. Verwendung finden.

Die erfindungsgemäss hergestellte Pflanzenextrakt-Brausezubereitung zeichnet sich durch eine schnelle Auflösung in Wasser ( Auflösezeit bei 17°C: 1 ½ bis 4 Minuten) und durch ein wesentlich verbessertes Schaumverhalten, d.h. nur geringes Schäumen, aus.

Die Erfindung wird im folgenden an Hand eines Beispieles näher erläutert. Weitere Beispiele für verschiedene Pflanzenextrakte werden in einer Tabelle zusammengefasst.

### Beispiel 1: Efeuextrakt-Brausetablette

### Herstellung der Efeuextrakt-Phase:

65 Gewichtsteile Efeu-Trockenextrakt werden auf etwa 45 - 50°C aufgeheizt. Der Trockenextrakt wird mit einer Lösung aus 5 Gewichtsteilen Simethicon, 2 Gewichts-teilen Capryl-caprinsäure-Triglicerid (Miglyol 812®), 0,2 Gewichtsteilen Tagat® R40 (ethoxylierter Glycerinfettsäureester), in 1,7 Gewichtsteilen Butanon und 0,7 Gewichtsteilen Ethanol 96%ig behandelt. Diese Lösung lässt man unter Rühren auf den Efeu-Trockenextrakt verteilen und fügt vor dem Trocknen 100 Gewichtsteile Mannitol zu. Anschliessend wird das Produkt mittels Vakuum unter langsamem Rühren getrocknet und die Phase auf 0,5 mm gesiebt.

In diesem Falle wird sowohl die ölige Substanz als auch der Emulgator und das Anti-Schaummittel Simethicon in einem Schritt auf den Efeu-Trockenextrakt aufgebracht.

Das Brausegranulat wird mit folgenden Inhaltsstoffen und Mengen hergestellt: 1165 Gewichtsteile Zitronensäure kristallin, 250 Gewichtsteile Zitronensäure Pulver, 3 Gewichtsteile Saccharin-Natrium und 50 Gewichtsteile Natriumcyclamat wurden auf 60°C aufgeheizt und mit einer Lösung aus 5 Gewichtsteilen Natriumcitrat und 5,5 Gewichtsteilen Wasser befeuchtet. Anschliessend fügt man 897 Gewichtsteile Natriumhydrogencarbonat zu und lässt kontrolliert reagieren. Vor dem Trocknen setzt man 88 Gewichtsteile Natriumcarbonat zu und trocknet das Produkt anschliessend mittels Vakuum bei einer Temperatur von über 50°C bis auf 15 mbar.

Für das pressfertige Granulat werden 172.2 Gewichtsteile der Pflanzenextrakt-Phase mit 2458 Gewichtsteilen einer Brausebasis sowie 200 Gewichtsteilen Sorbitol, 298 Gewichtsteilen Mannitol und mit 60 Gewichtsteilen Aroma sowie 210 Gewichtsteilen Maltodextrin vermischt und zu Tabletten von 3,4 g verpreßt. Für die Aromatisierung kann statt des Maltodextrin auch ein Fruchtpulver in der Grössenordnung von 250 bis 270 Gewichtsteilen zugesetzt werden

Das Produkt zeigt eine geringe Schaumbildung und eine Auflösezeit von 2½ bis maximal 3 Minuten, wobei eine vergleichbare Brausetablette ohne behandelten Pflanzenextrakt eine Auflösezeit von 5 bis 7 Minuten zeigt.

Die Beispiele 2 bis 17 mit weiteren leicht löslichen Pflanzenextrakten sind in den nachfolgenden Tabellen 1 bis 4 angeführt, wobei die Herstellung im wesentlichen der Herstellung des Beispiels 1 entspricht.

**Tabelle 1**

| **Belspiel Nr.** | 2 | 3 | 4 | 5 |
|---|---|---|---|---|
| | Efeu | Birkenblätter | Cynara | Thymian |
| **Brausebasis** | 73.60 | 63.80 | 84.79 | 73.63 |
| **Wirkstoffphase** | | | | |
| *Wirkstoff* | 1.99 | 11.80 | 14.62 | 8.57 |
| | | | | |
| *Fettig*/*Ölige*/*Wachsartige Substanz:* | | | | |
| Isopropylpalmitat | | | 0.46 | |
| Macrogolglycerolrizinoleat | | | | 0.57 |
| Capryl-Caprinsäure Triglycerid (Miglyol®) | 0.06 | | | |
| Polyethylenglykol (Carbowax) | | 0.78 | | |
| *Gew.% aufWirkstoffbezogen* | 3,02 | 6,61 | 3,15 | 6,65 |
| | | | | |
| *Emulgatoren:* Sorbitanmonolaurat | | | 0.05 | |
| Polyoxyethylenglycerol Fettsäureester (Tagat®) | 0.01 | | | |
| Sojalecithin + Mono- und Diglyceride (Metarin®) | | 0.10 | | 0.09 |
| *Gew. % auf Wirkstoff bezogen* | 0,50 | 0,85 | 0,34 | 1,05 |
| | | | | |
| *Anfischaummittel:* Simethicon | 0.15 | | | |
| *Gew. % auf Wirkstoff bezogen* | 7,54 | | | |
| | | | | |
| *Füllstoff:* Mannitol | 2.99 | 9.44 | | 5.71 |
| | | | | |
| **Zumischung** | | | | |
| *Emulgatorphase* Magnesiumstearat | | | 0.09 | |
| Mannitol | | 5.1 | | 5.60 |
| Docusat-Natrium | | 0.10 | | 0.11 |
| *EmulgatorGew.% auf Wirkstoff bezogen* | | 0,85 | 0,62 | 1,28 |
| | | | | |
| *Antischaumphase* Mannitol | | 5.21 | | 5.68 |
| Simethicon | | 0.02 | | 0.03 |
| *Antischaummittel:Gew.% auf Wirkstoff bezogen* | | 0,17 | | 0,35 |
| | | | | |
| *Füllstoffe* Mannitol | 8.93 | | | |
| Sorbitol | 5.99 | 3.64 | | |
| Maltodextrin | 6.29 | | | |
| **Summe** | 100 | 100 | 100 | 100 |
| **Gesamt (Gew.% auf Wirkstoff bezogen)** | | | | |
| *Fettig*/*Ölige*/*Wachsartige Substanz*: | 3,02 | 6,61 | 3,15 | 6,65 |
| *Emulgato:r* | 0,50 | 1,70 | 0,96 | 2,33 |
| *Antischaummittel:* | 7,54 | 0,17 | | 0,35 |
| | | | | |
| Tablettengewicht [mg] | 3340 | 1850 | 3480 | 3500 |
| Auflöszeit tsec] | 120 | 140 | 140 | 100 |

**Tabelle 2**

| **Beispiel Nr.** | 6 | 7 | 8 | 9 |
|---|---|---|---|---|
| | Plantago | Plantago | Fol.Urticae | Rad.Urticae |
| | | | | |
| **Brausebasis** | 76.63 | 68.88 | 61.75 | 86.89 |
| | | | | |
| **Wirkstoffphase** | | | | |
| | | | | |
| *Wirkstoff* | 19.53 | 20.87 | 10.93 | 10.00 |
| | | | | |
| *Fettig*/*Ölige*/*Wachsartige Substanz:* | | | | |
| Isopropylpalmitat | 0.51 | | | 0.07 |
| Macrogolglycerolrizinoleat | 0.11 | | 0.82 | |
| Tocopherolacetat | | 3.91 | | |
| Gew. % auf *Wirkstoff bezogen* | 3,17 | 18.74 | 7.50 | 0.70 |
| | | | | |
| *Emulgatoren:* Sorbitanmonolaurat | | | | 0.02 |
| Sojalecithin + Mono-& Diglyceride (Metarin®) | | | 0.11 | |
| *Gew.% auf Wirkstoff bezogen* | | | 1.01 | 0.20 |
| | | | | |
| *Füllstoff:* Mannitol | | | 10.00 | |
| Aerosil | 0.20 | | | |
| **Zumischung** | | | | |
| *Fettige*/*Ölige*/*Wachsige Phase* | | | | |
| Macrogolglycerolrizinoleat | 0.01 | | | 0.01 |
| *Träger:* Mannitol | 1.46 | | | 1.47 |
| Gew *% auf Wirkstoff bezogen* | 0.05 | | | 0.10 |
| | | | | |
| *Emulgatorphase* | | | | |
| Magnesiumstearat | 0.08 | 0.06 | | 0.06 |
| Docusat-Natrium | | 0.06 | 0.06 | |
| *Träger:* Mannitol | | 3.07 | 2.67 | |
| *Emulgator: Gew. % auf Wirkstoff bezogen* | 0.41 | 0.57 | 0.55 | 0.60 |
| | | | | |
| *Antischaumphase* Simethicon | 0.01 | 0.01 | 0.14 | 0.01 |
| *Träger:* Mannitol | 1.46 | 3.13 | 13.52 | 1.47 |
| *Gew.* % *auf Wirkstoff bezogen* | 0.05 | 0.05 | 1.28 | 0.10 |
| | | | | |
| **Summe** | 100 | 100 | 100 | 100 |
| | | | | |
| **Gesamt (Gew.% auf Wirkstoff bezogen)** | | | | |
| *Fettig*/*Ölige*/*Wachsartige Substanz:* | 3,22 | 18.74 | 7.50 | 0.80 |
| *Emulgator* | 0,41 | 0.57 | 1.56 | 0.80 |
| *Antischaummittel:* | 0.05 | 0,05 | 1.28 | 0.10 |
| | 100 | 100 | 100 | 100 |
| | | | | |
| Tablettengewicht [mg] | 3410 | 3110 | 3660 | 3400 |
| Auflöszeit [sec] | 110 | 120 | 100 | 100 |

**Tabelle 3**

| **Beispiel Nr.** | 10 | 11 | 12 | 13 |
|---|---|---|---|---|
| | Solidago | Solidago | Solidago | Solidago |
| | | | | |
| **Brausebasis** | 71.38 | 71.38 | 71.37 | 70.00 |
| | | | | |
| **Wirkstoffphase** | | | | |
| | | | | |
| *Wirkstoff* | 13.05 | 13.05 | 13.05 | 12.80 |
| | | | | |
| *Fettig*/*Ölige*/*Wachsartige Substanz:* | | | | |
| Capryl-Caprinsäure Triglycerid (Miglyol®) | 0.53 | 0.53 | 0.53 | 1.15 |
| Kokosfett gehärtet | | | 0.18 | 0.29 |
| Myristinsäuretriglycerid (Dynasan®) | | 0.18 | | |
| Hydriertes Rizinusöl (Cutina®) | 0.18 | | | |
| *Gew % auf Wirkstoff bezogen* | 5.44 | 5.44 | 5.44 | 11.25 |
| | | | | |
| *Emulgatoren* | | | | |
| Polyoxyethylenglycerol Fettsäureester (Tagat®) | 0.18 | | 0.18 | 0.29 |
| Sojalecithin | | 0.18 | | |
| Polyoxyethylen(20)sorbitanmonolaurat (Tween®) | | | 0.01 | |
| *Gew.* % *auf Wirkstoff bezogen* | 1.38 | 1.38 | 1.46 | 2.27 |
| | | | | |
| *Antischaummittel:* Simethicon | | | | 0.17 |
| *Gew. % auf Wirkstoff bezogen* | | | | 1.33 |
| | | | | |
| *Füllstoff:* Lactose | 11.75 | 11.75 | 11.75 | 5.76 |
| | | | | |
| **Zumischung** | | | | |
| | | | | |
| *Füllstoffe* Sorbitol | | | | 6.66 |
| Natriumsulfat | 2.94 | 2.94 | 2.94 | 2.88 |
| | | | | |
| **Summe** | 100 | 100 | 100 | 100 |
| | | | | |
| **Gesamt (Gew.% auf Wirkstoff bezogen)** | | | | |
| *Fettig*/*Ölige*/*Wachsartige Substanz:* | 5.44 | 5.44 | 5.44 | 11.25 |
| *Emulgato:r* | 1.38 | 1.38 | 1.46 | 2.27 |
| *Antischaummittel:* | | | | 1.33 |
| | | | | |
| Tablettengewicht [mg] | 3503 | 3503 | 3503 | 3340 |
| Auflöszeit [sec] | 210 | 210 | 220 | 165 |

**Tabelle 4**

| **Beispiel** | 14 | 15 | 16 | 17 |
|---|---|---|---|---|
| | Solidago | Solidago | Solidago | Solidago |
| **Brausebasis** | 70.20 | 56.36 | 82.18 | 53.67 |
| **Wirkstoffphase** | | | | |
| *Wirkstoff* | 12.83 | 10.30 | 12.58 | 12.80 |
| | | | | |
| *Fettig*/*Ölige*/*Wachsartige Substanz:* | | | | |
| Isopropylpalmitat | | | 0.69 | |
| Capryl-Caprinsäure Triglycerid (Miglyol®) | | 0.70 | | 0.87 |
| Kokosfett ungehärtet | 0.29 | | | |
| *Gew. % auf Wirkstoff bezogen* | 2.26 | 6.80 | 5.48 | 6.80 |
| | | | | |
| *Emulgatoren:* Sorbitanmonostearat | | | 0.07 | |
| Sorbitanmonoisostearat | | 0.05 | | |
| Natriumstearoyllactat | | | 0.01 | |
| Polyoxyethylenglycerol Fettsäureester/Tagat® | 0.29 | | | 0.87 |
| *Gew. % auf Wirkstoff bezogen* | 2.26 | 0.49 | 0.64 | 6.80 |
| | | | | |
| *Antischaummittel:* Simethicon | 0.17 | 0.19 | 0.14 | |
| *Gew.* % *auf Wirkstoff bezogen* | 1.33 | 1.84 | 1.11 | |
| | | | | |
| *Füllstoff:* Lactose | 5.78 | 4.64 | | 8.67 |
| Sorbitol | | 0.23 | | |
| **Zumischung** | | | | |
| *Fettig*/*Ölige*/*Wachsige Phase* | | | | |
| Macrogolglyceroirhinzinoleat | | | 0.04 | |
| *Träger:* Mannitol | | | 4.21 | |
| *Gew.* % *auf Wirkstoff bezogen* | | | 0.34 | |
| | | | | |
| *Emulgator:* Magnesiumstearat | | | 0.09 | |
| Gew % *auf Wirkstoff bezogen* | | | 0.72 | |
| | | | | |
| *Antischaumphase* Simethicon | | 0.12 | | 0.12 |
| *Träger:* Mannitol | | 11.48 | | 14.33 |
| *Antischaummittel: Gew.* % *auf Wirkstoff bezogen* | | 1,17 | | 0,94 |
| *Füllstoffe* Mannitol | | 8.12 | | |
| Sorbitol | 7.54 | | | 5.78 |
| Lactose | | 7.82 | | |
| Natriumsulfat | 2.89 | | | 2.89 |
| | | | | |
| **Summe** | 100 | 100 | 100 | |
| **Gesamt (Gew.% auf Wirkstoff bezogen)** | | | | |
| *Fettig*/*Ölige*/*Wachsartige Substanz:* | 2.26 | 6.80 | 5.82 | 6,80 |
| *Emulgator* | 2.26 | 0.49 | 1.36 | 6.80 |
| *Antischaummittel:* | 1.33 | 3.01 | 1.11 | 0,94 |
| | | | | |
| Tablettengewicht [mg] | 3460 | 4310 | 3533 | 3504 |
| Auflöszeit [sec] | 230 | 115 | 145 | 150 |

Solidago- und Birkenblätterextrakte sind beide stark saponinhältig, erfordern aber trotzdem eine unterschiedliche Fett- und Emulgator-Phase. Während bei den Birkenblättem vorzugsweise Carbowax 400 als fettige Substanz und Metharin (ein Phospholipid) als Emulgator eingesetzt werden, haben beim Solidagoextrakt Miglyol als fettige Substanz und Sorbitan-Mono-Isostearat als Emulgator die besten Ergebnisse gezeigt. Bei diesen Beispielen kommen neben der Schaumbildung auch die Auflösungseigenschaften zum Tragen, d.h. beim Birkenblätterextrakt steht vorrangig die Schaumbekämpfung im Vordergrund, da die Birkenblätterextrakte die Auflösezeit nicht in dem starken Masse verlängert haben, wie es beim Solidago der Fall ist. Beim Solidagoextrakt steht neben der Schaumbekämpfung primär die Verkürzung der Auflösezeit von nahezu 20 Minuten auf unter4 Minuten im Vordergrund, was durch die beschriebene Massnahme bewirkt werden konnte. Fast jeder Extrakt besitzt - bedingt durch die jeweilige Vielzahl von Inhaltstoffen - ein ganz eigenes Verhalten, so dass das Optimum auf Basis der gegebenen Informationen jeweils eingestellt werden muss.

Brausetabletten, die nicht erfindungsgemäss behandelt bzw. hergestellt wurden, beginnen im Wasser an der Oberfläche anfangs zu brausen; der Brauseeffekt und damit die Auflösung werden aber zunehmend durch die Bildung einer hochkonzentrierten, klebrig-schleimigen Lösung zwischen den Brausegranulaten gebremst. Damit wird der weitere Zutritt von Wasser in den Kern der Brausetablette verhindert, sodass dieser trocken bleibt und die Auflösung entsprechend langsam erfolgt.

## Patentansprüche

1. Brausezubereitung in Form von Granulat oder einer Tablette, enthaltend wenigstens einen wasserlöslichen oder zumindest suspendierbaren Pflanzenextrakt und eine Brausebasis aus wenigstens einer festen, essbaren, organischen Säure und wenigstens einem Alkali- und/oder Erdalkalicarbonat bzw. -hydrogencarbonat, **dadurch gekennzeichnet, dass** die Pflanzenextraktteilchen von wenigstens einer öligen, fettigen oder wachsartigen Substanz überzogen sind.

2. Brausezubereitung nach Anspruch 1, **dadurch gekennzeichnet, dass** der Pflanzenextrakt wenigstens von einer der Pflanzen bzw. Pflanzenteile aus der Gruppe Efeu, Sabale, Solidago, Plantago, Brennesselwurzel, Brennesselblätter, Birkenblätter, Cynara, Thymian, Johanniskraut, Harpagophytum, Gingko und Ruscus aculeatus stammt.

3. Brausezubereitung nach Anspruch 1 oder 2, **dadurch gekennzeichnet, dass** die ölige, fettige oder wachsartige Substanz wenigstens eine Substanz aus der Gruppe bestehend aus Neutralölen, wie Glycerin bzw. Propylenglykolestern der Capryl- und Caprinsäure; essbaren, tierischen oder pflanzlichen Fetten, insbesondere mikrokristallinen Triglyceriden und Glycerinestern gesättigter, geradzahliger und unverzweigter höherer Fettsäuren, etwa der Grössenordnung C₁₀ - C₂₂; gehärtetem Kokosfett; hydriertem Rizinusöl; Tocopherolacetat; Estern höherer Fettsäuren, wie z.B. Jsopropylpalmitat; und Polyethylenglykolen umfasst und bevorzugt in einer Menge von 0,5 - 25, insbesondere von 0,8 - 19 Gewichtsteilen (auf 100 Gewichtsteile Pflanzenextrakt bezogen) vorliegt.

4. Brausezubereitung nach einem der vorhergehenden Ansprüche, **dadurch gekennzeichnet, dass** sie ausserdem - bevorzugt 0,05 bis 0,5, insbesondere 0,1 - 0,4 Gewichtsteile - auf 100 Gewichtsteile Pflanzenextrakt bezogen - der öligen, fettigen oder wachsartigen Substanz, vorzugsweise auf einem Träger, in Mischung mit den anderen Bestandteilen enthält.

5. Brausezubereitung nach einem der vorhergehenden Ansprüche, **dadurch gekennzeichnet, dass** sie ausserdem - bevorzugt 0,2 - 10, insbesondere 0,3 - 8 Gewichtsteile (auf 100 Gewichtsteile des Pflanzenextraktes bezogen) - eines Emulgators enthält, insbesondere wenigstens eine Substanz aus der Gruppe von Phospholipiden, Polysorbaten, ethoxylierten Glycerinfettsäureestern, Zuckerestern, Glycerin-Polyethylenglykol-Oxystearaten, Macrogol-Glycero-Rizinoleat, Natriumstearyllactat und Lipid-Emulgatoren, wie insbesondere Propylenglycolstearat, Glycerinoleat, -laurat und -stearat.

6. Brausezubereitung nach Anspruch 5, **dadurch gekennzeichnet, dass** der Emulgator wenigstens teilweise in dem Überzug des Pflanzenextraktes enthalten ist, bzw. die allenfalls restliche Emulgatormenge auf einem Träger in einer Menge von 0,3 - 3,0, insbesondere 0,4 - 1,5 Gewichtsteilen (auf 100 Gewichtsteile des Pflanzenextraktes bezogen) aufgebracht und mit den anderen Bestandteilen gemischt vorliegt.

7. Brausezubereitung nach einem der vorhergehenden Ansprüche, **dadurch gekennzeichnet, dass** die Gesamtmenge der öligen, fettartigen oder wachsartigen Substanz einschlisslich allfälliger Emulgatoren 0,5 - 35, insbesondere 1,0 - 27 Gewichtsteile (auf 100 Gewichtsteile des Pflanzenextraktes bezogen) beträgt.

8. Brausezubereitung nach einem der vorhergehenden Ansprüche, **dadurch gekennzeichnet, dass** sie ausserdem - bevorzugt 0,05 - 10, insbesondere 0,6 - 3,0 Gewichtsteile (auf 100 Gewichtsteile des Pflanzenextraktes bezogen) - eines Anti-Schaummittels enthält, insbesondere Dimethicon oder Simethicon.

9. Brausezubereitung nach Anspruch 8, **dadurch gekennzeichnet, dass** das Anti-Schaummittel im Überzug enthalten und/oder auf einem Träger - insbesondere in einer Menge von etwa 0,06 - 1,5 Gewichtsteilen (auf 100 Gewichtsteile des Pflanzenextraktes bezogen) - aufgebracht mit den anderen Bestandteilen gemischt vorliegt.

10. Brausezubereitung nach einem der vorhergehenden Ansprüche, **dadurch gekennzeichnet, dass** sie ausserdem wenigstens einen Zusatz- und/oder Hilfsstoff aus der Gruppe pharmazeutisch zulässiger Füllstoffe, wie Zucker; Zuckeralkohole, Hydrokolloide, Aromastoffe; künstliche Süssstoffe und Tenside enthält.

11. Verfahren zur Herstellung einer Pflanzenextrakt Phase für eine Brausezubereitung nach einem der vorhergehenden Ansprüche, **dadurch gekennzeichnet, dass** der Pflanzenextrakt - vorzugsweise in einem Granulator, insbesondere in einem Vakuumgranulator - erwärmt und mit einer Schmelze oder Lösung der öligen, fettigen oder wachsartigen Substanz benetzt oder versetzt, anschliessend - vorzugsweise im Vakuum - getrocknet und auf die gewünschte Körnung gesiebt wird.

12. Verfahren nach Anspruch 11, **dadurch gekennzeichnet, dass** der Schmelze bzw. Lösung wenigstens ein Emulgator und/oder wenigstens ein Antischaummittel zugesetzt wird.

13. Verfahren zur Herstellung einer Füllstoff-Phase für eine Brausezubereitung nach einem der Ansprüche 1 bis 9, **dadurch gekennzeichnet, dass** ein pharmazeutisch zulässiger Füllstoff erwärmt und - vorzugsweise in einem Granulator, insbesondere einem Vakuumgranulator - mit einer Schmelze oder Lösung der öligen, fettigen oder wachsartigen Substanz und/oder wenigstens eines Emulgators und/oder wenigstens eines Anti-Schaummittels benetzt oder versetzt, anschliessend - vorzugsweise im Vakuum - getrocknet und auf die gewünschte Körnung gesiebt wird.

## Claims

1. Effervescent composition in the form of granulated material or of a tablet, containing at least one water-soluble or at least suspendable plant extract and an effervescent base comprising at least one solid, edible organic acid and at least one alkali metal and/or alkaline earth metal carbonate and/or bicarbonate, **characterized in that** the plant extract particles are coated by at least one oily, fatty or waxy substance.

2. Effervescent composition as claimed in Claim 1, **characterized in that** the plant extract originates at least from one of the plants or plant parts from the group consisting of ivy, sabale, solidago, plantago, stinging nettle root, stinging nettle leaves, birch leaves, cynara, thyme, St. John's Wort, harpagophytum, gingko and Ruscus aculeatus.

3. Effervescent composition according to Claim 1 or 2, **characterized in that** the oil, fatty or waxy substance comprises at least one substance consisting of neutral oils, such as glycerol or propylene glycol esters of caprylic and capric acid; edible, animal or vegetable fats, in particular microcrystalline triglycerides and glyceryl esters of saturated, even-numbered and straight-chain higher fatty acids, for example of the order of magnitude of C₁₀-C₂₂; hydrogenated coconut oil; hydrogenated castor oil; tocopheryl acetate; esters of higher fatty acids, such as, for example, isopropyl palmitate; and polyethylene glycols, and is preferably present in an amount of 0.5 - 25, in particular of 0.8 - 19, parts by weight (based on 100 parts by weight of plant extract).

4. Effervescent composition according to any of the preceding Claims, **characterized in that** it furthermore contains preferably from 0.05 to 0.5, in particular 0.1 - 0.4, part by weight - based on 100 parts by weight of plant extract - of the oily, fatty or waxy substance, preferably on a carrier, as a mixture with the other ingredients.

5. Effervescent composition according to any of the preceding Claims, **characterized in that** it furthermore contains preferably 0.2 - 10, in particular 0.3 - 8, parts by weight (based on 100 parts by weight of the plant extract) of an emulsifier, in particular at least one substance from the group consisting of phospholipids, polysorbates, ethoxylated glyceryl fatty esters, sugar esters, glyceryl polyethylene glycol oxystearates, macrogol glyceroricinoleate, sodium stearyl lactate and lipid emulsifiers, such as, in particular, propylene glycol stearate and glyceryl oleate, laurate and stearate.

6. Effervescent composition according to Claim 5, **characterized in that** the emulsifier is contained at least partly in the coating of the plant extract, and any residual amount of emulsifier is applied to a carrier in an amount of 0.3 - 3.0, in particular 0.4 - 1.5, parts by weight (based on 100 parts by weight of the plant extract) and is present as a mixture with the other ingredients.

7. Effervescent composition according to any of the preceding Claims, **characterized in that** the total amount of the oily, fatty or waxy substance, including any emulsifiers, is 0.5 - 35, in particular 1.0 - 27, parts by weight (based on 100 parts by weight of the plant extract).

8. Effervescent composition according to any of the preceding Claims, **characterized in that** it furthermore contains preferably 0.05 - 10, in particular 0.6 - 3.0, parts by weight (based on 100 parts by weight of the plant extract) of an antifoam, in particular dimethicone or simethicone.

9. Effervescent composition according to Claim 8, **characterized in that** the antifoam is contained in the coating and/or is applied to a carrier - in particular in an amount of about 0.06 - 1.5 parts by weight (based on 100 parts by weight of the plant extract) - as a mixture with the other ingredients.

10. Effervescent composition according to any of the preceding Claims, **characterized in that** it furthermore contains at least one additive and/or excipient from the group consisting of pharmaceutically permissible fillers, such as sugar; sugar alcohols, hydrocolloids, flavours; artificial sweeteners and surfactants.

11. Process for the preparation of a plant extract phase for an effervescent composition according to any of the preceding Claims, **characterized in that** the plant extract is heated - preferably in a granulator, in particular in a vacuum granulator - and is wet or mixed with a melt or solution of the oily, fatty or waxy substance, then dried - preferably in vacuo - and sieved to the desired particle size.

12. Process according to Claim 11, **characterized in that** at least one emulsifier and/or at least one antifoam is added to the melt or solution.

13. Process for the preparation of a filler phase for an effervescent composition according to any of Claims 1 to 9, **characterized in that** a pharmaceutically permissible filler is heated and - preferably in a granulator, in particular a vacuum granulator - is wet or mixed with a melt or solution of the oily, fatty or waxy substance and/or at least one emulsifier and/or at least one antifoam, then dried-preferably in vacuo - and sieved to the desired particle size.

## Revendications

1. Composition effervescente sous la forme de granulés ou de comprimés contenant au moins un extrait végétal hydrosoluble ou du moins susceptible d'être mis en suspension dans de l'eau et une base effervescente constituée par au moins un acide organique solide alimentaire et par au moins un carbonate ou un hydrogénocarbonate d'un métal alcalin et / ou alcalino-terreux, **caractérisée en ce que** les particules de l'extrait végétal sont recouvertes par au moins une substance huileuse, grasse ou cireuse.

2. Composition effervescente selon la revendication 1, **caractérisée en ce que** l'extrait végétal provient d'au moins une des plantes ou d'une partie des plantes choisies dans le groupe suivant : lierre, Sabal, Solidago, Plantago, racines d'ortie, feuilles d'ortie, feuilles de bouleau, Cynara, thym, millepertuis, Harpagophytum, Gingo et Ruscus aculeatus.

3. Composition effervescente selon la revendication 1 ou la revendication 2, **caractérisée en ce que** la substance huileuse, grasse ou cireuse comprend au moins une substance choisie dans le groupe constitué par les huiles neutres comme la glycérine ou les esters de propylène glycols et des acides caprylique et décanoïque ; les matières grasses alimentaires animales ou végétales, en particulier les triglycérides microcristallins et les esters microcristallins du glycérol et d'acides gras supérieurs saturés à chaînes droites et non ramifiées, ayant une longueur de l'ordre de C₁₀ - C₂₂ ; la matière grasse durcie de noix de coco ; l'huile de ricin hydrogénée ; l'acétate de tocophérol ; les esters d'acides gras supérieurs comme par exemple le palmitate d'isopropyle ; et les polyéthylène glycols, dans une proportion allant, de préférence, de 0,5 à 25 et, plus particulièrement, de 0,8 à 19 parties en poids pour 100 parties en poids d'extrait végétal.

4. Composition effervescente selon l'une des revendications précédentes, **caractérisée en ce qu'**elle contient, en outre, pour 100 parties en poids d'extrait végétal, de préférence de 0,05 à 0,5 et, en particulier, de 0,1 à 0,4 parties en poids de substance huileuse, grasse ou cireuse, de préférence sur un support, en mélange avec les autres composants.

5. Composition effervescente selon l'une des revendications précédentes, s **caractérisée en ce qu'**elle contient, en outre, pour 100 parties en poids d'extrait végétal, de préférence de 0,2 à 10 et, en particulier, de 0,3 à 8 parties en poids d'un émulsifiant, en particulier d'au moins une substance choisie dans le groupe constitué par les phospholipides, les polysorbates, les esters éthoxylés du glycérol et d'acides gras, les esters de sucres, les oxystéarates du glycérol-polyéthylène glycol, le ricinoléate de macrogol-glycérol, le stéaryl-lactate de sodium et des émulsifiants lipidiques, comme en particulier le stéarate du propylène glycol, et l'oléate, le laurate et le stéarate de glycérol

6. Composition effervescente selon la revendications 5, **caractérisée en ce que** l'émulsifiant est présent au moins partiellement dans le revêtement de l'extrait végétal, le restant éventuel de l'émulsifiant étant appliqué sur un support à raison de 0,3 à 3,0 et, en particulier, de 0,4 à 1,5 parties en poids (pour 100 parties en poids de l'extrait végétal), en mélange avec les autres composants.

7. Composition effervescente selon l'une des revendications précédentes, **caractérisée en ce que** la quantité totale de la substance huileuse, grasse ou cireuse et celle de l'émulsifiant éventuel, est de 0,5 à 35 et, en particulier, de 1,0 à 27 parties en poids pour 100 parties en poids de l'extrait végétal.

8. Composition effervescente selon l'une des revendications précédentes, **caractérisée en ce qu'**elle contient, en outre, pour 100 parties en poids d'extrait végétal, de préférence de 0,05 à 10 et, en particulier, de 0,6 à 3,0 parties en poids d'un agent antimousse, en particulier de Dimethicon ou de Simethicon.

9. Composition effervescente selon la revendication 8, **caractérisée en ce que** l'agent antimousse contenu dans le revêtement et/ ou sur un support est présent en particulier à raison de 0,06 à 1,5 parties en poids (pour 100 parties en poids de l'extrait végétal), en mélange avec les autres composants.

10. Composition effervescente selon l'une des revendications précédentes, **caractérisée en ce qu'**elle contient, en outre, au moins un additif et /ou adjuvant choisi dans le groupe des charges acceptables sur le plan pharmaceutique, telles que les sucres ; les sucres alcools, les hydrocolloïdes, les agents aromatisants ; les édulcorants de synthèse et les tensioactifs.

11. Procédé pour préparer une phase d'extrait végétal pour une composition effervescente selon l'une des revendications précédentes, **caractérisé en ce que** l'extrait végétal est chauffé, de préférence, dans un appareil de granulation et, en particulier, dans un appareil de granulation à vide, puis mouillé ou mélangé avec une substance huileuse, grasse ou cireuse, liquide ou en solution et, ensuite, le tout est séché - de préférence sous vide - et tamisé pour avoir la granulométrie souhaitée.

12. Procédé selon la revendication 11, **caractérisé en ce que** l'on ajoute au liquide ou à la solution au moins un émulsifiant et / ou au moins un agent antimousse.

13. Procédé pour préparer une phase de charge pour une composition effervescente selon l'une des revendications 1 à 9, **caractérisé en ce qu'**un additif acceptable sur le plan pharmaceutique est chauffé, de préférence, dans un appareil de granulation et, en particulier, dans un appareil de granulation sous vide et mouillé ou mélangé avec la substance huileuse, grasse ou cireuse, liquide ou en solution, et / ou avec au moins un émulsifiant et / ou avec au moins un agent antimousse, puis le tout est séché - de préférence sous vide - et tamisé pour avoir la granulométrie souhaitée.
